# EUROPEAN PATENT APPLICATION

(11) **EP 2 269 549 A1**
(43) Date of publication of application: **05.01.2011**
(21) Application number: 09725434.6
(22) Date of filing: 16.01.2009
(51) Int. Cl.: A61F 13/15, A61F 13/49, A61F 13/494, A61F 13/511

(54) **ABSORPTIVE ARTICLE**

(30) Priority: 25.03.2008 JP 2008078893
(71) Applicant: Unicharm Corporation, Ehime 799-0111 (JP)
(72) Inventor: MINATO, Hironao, Kanonji-shi Kagawa 769-1602 (JP); NITTA, Reiko, Kanonji-shi Kagawa 769-1602 (JP)
(74) Representative: Stark, Gordon Drummond
(86) International application number: PCT/JP2009/050583
(87) International publication number: WO 2009/119135

(57) **Abstract**

An absorbent article provides on the side of a chassis 2 facing the wearer's skin, a separator sheet 3 adapted to be spaced from the chassis 2. The separator sheet 3 is formed in a middle zone 32 thereof with a first lotion-coated region 51 and a liner 26 provided on the side of a liquid-absorbent panel 23 facing the wearer's skin is formed with a second lotion-coated region 52. The front and rear lotion-coated sub-regions 52a, 52b are spaced from each other by the intermediary of the first lotion-coated region 51. The lotion is applied from the side facing the wearer's skin to the liquid-absorbent panel 23 with the separator sheet 3 laminated thereon so that the first and second lotion-coated regions 51, 52 may be simultaneously formed and the liquid-absorbent panel 23 is formed on a region thereof opposed to the middle zone 32 of the separator sheet 3 with a lotion-uncoated region 53.

## Description

### TECHNICAL FIELD

The present invention relates generally to absorbent articles and particularly to absorbent articles such as disposable diapers, toilet-training pants or incontinent briefs.

### RELATED ART

Absorbent articles implemented in disposable diapers are well known, for example, from the disclosure of JP 2004-298643 A (PATENT DOCUMENT 1). According to the disclosure of JP 2004-298643 A, the diaper comprises an inner sheet, an outer sheet and an absorbent structure sandwiched between these sheets. The inner sheet is coated on its side facing the wearer's skin with lotion functioning to alleviate a friction between the diaper and the wearer's skin and thereby to prevent the wearer from suffering various kinds of skin troubles. Furthermore, lotion coated on the inner sheet may be transferred to the wearer's skin as the inner sheet comes in contact with the wearer's skin and in consequence the wearer's skin may be coated with lotion. Lotion transferred to the wearer's skin serves as a barrier adapted to prevent the wearer's skin from being soiled with feces.
PATENT DOCUMENT 1: JP 2004-298643 A

### DISCLOSURE OF THE INVENTION

### PROBLEM TO BE SOLVED BY THE INVENTION

However, the inner sheet coated with lotion may have it potential liquid-permeability deteriorated due to the presence of lotion coated thereon. Particularly when the inner sheet has been coated over a wide range with such lotion, bodily fluids might not be able to permeate the inner sheet and might flow on the inner sheet, causing bodily fluids to leak out of the diaper. It is not ensured that the inner sheet comes in contact with the wearer' skin and, in this case, it is impossible to transfer lotion from the inner sheet to the wearer's skin. Unless lotion is reliably transferred to the wearer's skin, the function of lotion to prevent the wearer's skin from being soiled with feces is not expected.

It is an object of the invention to provide an absorbent article improved so as to ensure that lotion coated on the inner sheet would not cause leak of bodily fluids, and to ensure that this lotion can be reliably transferred to wearer's skin.

### MEASURE TO SOLVE THE PROBLEM

According to the present invention, there is provided an improvement of an absorbent article comprising a chassis having longitudinal and transverse directions, sides facing the skin of a wearer and a garment of the wearer, respectively, a front waist region, a rear waist region, a crotch region continuously extending between the front and rear waist regions, and a liquid-absorbent structure provided on at least the crotch region, and a separator sheet provided on the side of the chassis facing the wearer's skin and elastically biased to be spaced from the chassis in the crotch region, wherein the chassis is formed with a waist-opening defined by the front and rear waist regions and with a pair of leg-openings in the crotch region.

The improvement according to the present invention is characterized in that the separator sheet comprises lateral zones extending the longitudinal direction and opposite in the transverse direction, a middle zone connected between the lateral zones, through-holes defined by the lateral zones and the middle zone so as to communicate a side facing the wearer's skin with a side facing the chassis, a separator sheet biasing elastic members extending in the longitudinal direction and attached under tension to the separator sheet, and front and rear ends opposite in the longitudinal direction and extending in the transverse direction along which the separator sheet is permanently bonded to the side of the chassis facing the wearer's skin so that a void space is formed between the separator sheet and the liquid-absorbent structure and at least the middle zone comes in contact with the wearer's skin as the liquid-absorbent structure bows about the crotch region so that the front and rear waist regions are opposed to each other. The separator sheet is coated in the middle zone with lotion so as to form a first lotion-coated region and the chassis is coated on portions exposed through the through-holes with the lotion so as to form a second lotion-coated region.

According to one preferred embodiment of the invention, the chassis is provided in the crotch region with a lotion-uncoated region which is not coated with the lotion and the lotion-uncoated region is formed in a region of the chassis opposed to the middle zone of the separator sheet.

According to another preferred embodiment of the invention, the lotion is applied from the side of the separator sheet facing the wearer's skin toward the chassis.

According to still another preferred embodiment of the invention, the separator sheet includes a front through-hole lying on the side of the front waist region and a rear through-hole lying on the side of the rear waist region wherein the middle zone is provided between the front and rear through-holes and in the crotch region.

According to yet another preferred embodiment of the invention, the absorbent article further includes a third lotion-coated region formed on the side of the chassis facing the wearer's skin in at least one of the front and rear waist regions.

According to further another preferred embodiment of the invention, the third lotion-coated region is formed so as to extend in the transverse direction along the waist-opening.

According to an alternative preferred embodiment of the invention, the lotion consists of an emollient ingredient and an immobilizing ingredient serving to immobilize the emollient ingredient to the separator sheet and the chassis.

### EFFECT OF THE INVENTION

The separator sheet is coated in the middle zone thereof with the lotion so as to form the first lotion-coated region and this middle zone is adapted to come in contact with the wearer's skin during use of the diaper. In this way, the lotion is reliably transferred from the middle zone to the wearer's skin and the lotion transferred in this manner protects the wearer's skin from soiled with body wastes such as feces. The portions of the chassis exposed through the through-holes are also coated with the lotion so as to the second lotion-coated region. More specifically, the chassis is coated with the lotion not over the entire area thereof but over limited area thereof, i.e., only over the portions thereof exposed through the through-holes. Consequentially, such regions of the chassis left uncoated with the lotion well maintain the bodily fluid-perviousness required for the chassis and prevent leak of bodily fluids.

Such lotion-uncoated region formed in the chassis is defined by the portion of the chassis opposed to the middle zone of the separator sheet. This portion of the chassis opposed to the middle zone of the separator sheet so as to define the lotion-uncoated region never comes in contact with the wearer's skin since the middle zone opposed to this lotion-uncoated region faces the wearer's skin. In other words, there is no possibility that the lotion-uncoated sheet might come in contact with the wearer's skin and cause various kinds of skin troubles.

According to the embodiment wherein the lotion is applied from the side of the separator sheet facing the wearer's skin toward the chassis, the first lotion-coated region of the separator sheet, and the second lotion-coated region and the lotion-uncoated region of the chassis can be formed at once. As a result, the manufacturing cost which otherwise would be substantially increased due to the process of the lotion application can be efficiently reduced.

According to the embodiment wherein the separator sheet includes a front through-hole lying on the side of the front waist region and a rear through-hole lying on the side of the rear waist region wherein the middle zone is provided between the front and rear through-holes and the crotch region, the separator sheet is prevented from disturbing urination as well as defecation. The middle zone defined in the crotch region comes in contact with a region of the wearer's skin extending between the external genital and the anus, i.e., the region sensitive to irritation and apt to suffer from skin troubles such as eruption. The lotion transferred to such region of skin sensitive to irritation serves to protect the wearer's skin from various skin troubles.

According to the embodiment further including a third lotion-coated region formed on the side of the chassis facing the wearer's skin in at least one of the front and rear waist regions, it is possible to protect the wearer's skin from various kinds of skin trouble also in the waist regions.

According to the embodiment wherein the third lotion-coated region is formed so as to extend in the transverse direction along the waist-opening, the waist-opening is always held in contact with the wearer's skin during use of the wearing article and therefore the third lotion-coated region also held in contact with the wearer's skin substantially always. In a consequence, it is reliably possible to protect the wearer's skin in his or her waist regions from various kinds of skin troubles.

According to the embodiment wherein the lotion consists of an emollient ingredient and an immobilizing ingredient serving to immobilize the emollient ingredient to the separator sheet and the chassis, it is possible to emollient the wearer's skin and thereby to prevent the wearer's skin from suffering from various kinds of skin troubles.

### BRIEF DESCRIPTION OF THE DRAWINGS

[FIG. 1] Perspective view showing the diaper according to a first embodiment.
[FIG. 2] Sectional view taken along the line II-II in Fig. 1.
[FIG. 3] Plan view of the diaper.
[FIG. 4] Explanatory diagram of the diaper.
[FIG. 5] Explanatory diagram of the diaper.
[FIG. 6] View similar to Fig. 3, showing the diaper according to a second embodiment.

### IDENTIFICATION OF REFERENCE NUMERALS USED IN THE DRAWINGS

- 1: diaper
- 2: chassis
- 3: separator sheet
- 5: inner side facing the wearer's skin
- 6: outer side facing a garment of the wearer
- 7: front waist region
- 8: rear waist region
- 9: crotch region
- 10: liquid-absorbent structure
- 18: waist-opening
- 19: leg-opening
- 21: leg elastic member
- 23: liquid-absorbent panel
- 29: void space
- 30: lateral zone
- 31: lateral zone
- 32: middle zone
- 33: front through-hole
- 34: rear through-hole
- 51: first lotion-coated region
- 52: second lotion-coated region
- 53: lotion-uncoated region
- 54: front waist sheet
- 55: rear waist sheet
- 56: third front lotion-coated region (third lotion-coated region)
- 57: third rear lotion-coated region (third lotion-coated region)

### DETAILED DESCRIPTION OF THE PREFERRED EMBODIMENTS

Details of the present invention will be exemplarily described with reference to the accompanying drawings on the basis of a disposable diaper as one of embodiments of the present invention.

### <First Embodiment>

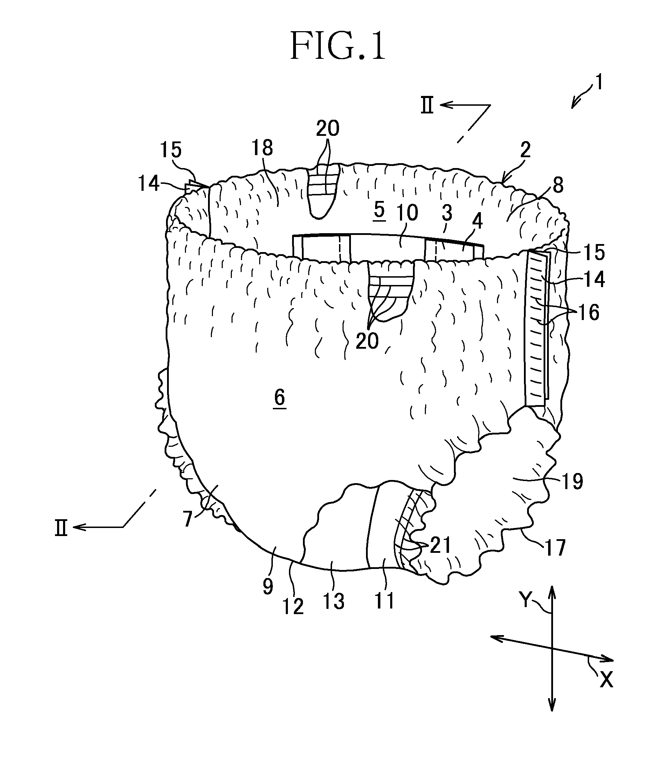

Figs. 1 through 5 illustrate a first embodiment of the invention. Fig. 1 shows a diaper 1 as put on the wearer's body. As will be apparent from Fig. 1, the diaper 1 comprises a chassis 2, a separator sheet 3 and leak-barrier cuffs 4. The chassis 2 is pants-shaped and comprises an inner side 5 facing the wearer's skin and an outer side 6 facing a garment of the wearer, a front waist region 7, a rear waist region 8 and a crotch region 9 extending between these front and rear waist regions 7, 8. A direction extending from the front waist region 7 across the crotch region 9 to the rear waist region 8 is referred to herein as a longitudinal direction Y and a direction extending orthogonally to this is referred to herein as a transverse direction X. The chassis 2 comprises the inner and outer sheets 11, 12, a liquid-impervious leak-barrier sheet 13 sandwiched between these inner and outer sheets 11, 12 and a liquid-absorbent structure 10 laminated on the inner sheet 11.

Opposite side edges 14 of the front waist region 7 and opposite side edges 15 of the rear waist region 8 are put flat and joined together at a plurality of joint streaks 16 arranged intermittently along the respective side edges 14, 15 to form both side seams. In this way, the chassis 2 of pull-on pants type is obtained. Being joined together at the joint streaks 16, a waist-opening 18 surrounded by the front and rear waist regions 7, 8 is formed and simultaneously a pair of leg-openings 19 respectively having peripheral edges 17. A plurality of waist elastic members 20 extend along the peripheral edge of the waist-opening 18 and a plurality of leg elastic members 21 extend the peripheral edges of the respective leg-openings 19. These elastic members 20, 21 are sandwiched between the inner sheet 11 and the outer sheet 12 and bonded under tension to at least one of these inner and outer sheets 11, 12 by adhesive (not shown).

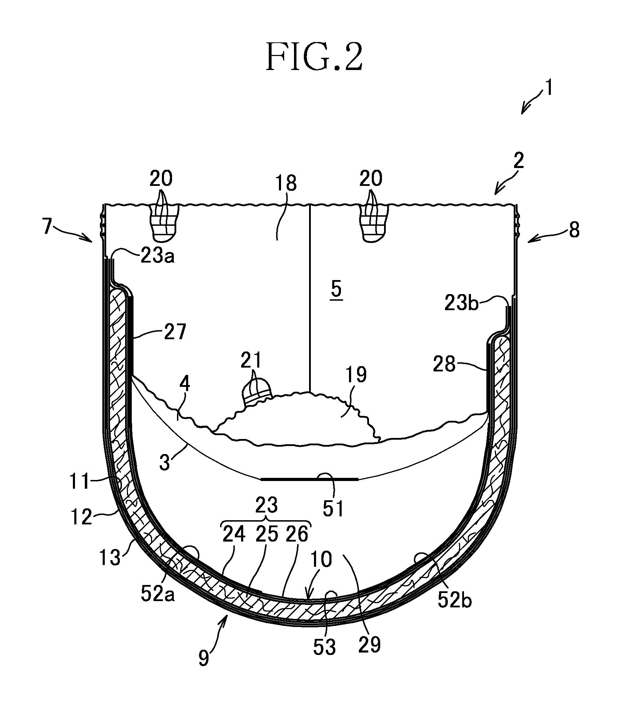

Fig. 2 is a sectional view taken along the line II-II in Fig. 1. As will be apparent from Fig. 2, a liquid-absorbent panel 23 constituting the liquid-absorbent structure 10 is provided on the inner side 5 of the inner sheet 11 facing the wearer's skin, the separator sheet 3 is provided aside from the liquid-absorbent structure 10 toward the wearer's skin and the leak-barrier cuffs 4 are provided aside from the separator sheet 3 toward the wearer's skin. The liquid-absorbent panel 23 has front and rear ends 23a, 23b opposed to each other in the longitudinal direction Y and extending in the transverse direction X.

The liquid-absorbent panel 23 extends in the longitudinal direction Y across the crotch region 9 and has its front and rear ends 23a, 23b bonded to the inner sheet 11 in the front and rear waist regions 7, 8. The liquid-absorbent panel 23 comprises a liquid-absorbent core 25 wrapped with a liquid-absorbent/dispersant sheet 24 such as tissue paper which is, in turn, covered with a liner 26 facing the wearer's skin. A side of the liquid-absorbent panel 23 facing the wearer's clothes is covered with the leak-barrier sheet 13 provided on the inner side of the outer sheet 12 so that bodily fluids once absorbed by the core 25 is restrained from leaking out from the diaper 1. If it is desired, the leak-barrier sheet 13 may be bonded directly to the bottom surface of the liquid-absorbent panel 23.

The separator sheet 3 is joined in the vicinity of its front and rear ends 27, 28 to the liner 26 facing the wearer's skin by adhesion or welding. The front and rear ends 27, 28 respectively lie in the front and rear waist regions 7, 8. In a pull-on pants-shaped state as illustrated, the separator sheet 3 is spaced upward from the liner 26 facing the wearer's skin as a result of bowing of the crotch region 9 and contraction of separator sheet biasing elastic members which will be described later. With the front and rear ends 27, 28 of the separator sheet 3 being joined to the liner 26 facing the wearer's skin, the separator sheet 3 as a whole looks now like a hammock slung. Consequentially, a void space 29 is defined between the separator sheet 3 and the liquid-absorbent panel 23.

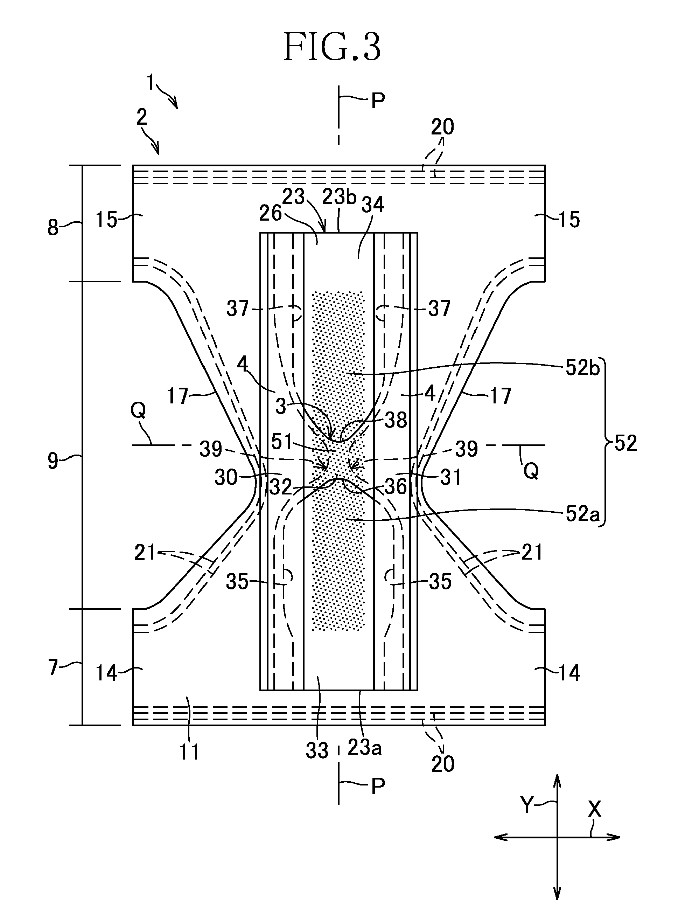

Fig. 3 is a plan view of the diaper 1 after the front and rear waist regions 7, 8 have been peeled off from each other at the joint streaks 16 shown in Fig. 1 and then has been developed and flattened in the longitudinal direction Y as well as in the transverse direction X so that the front and rear waist regions 7, 8 and the crotch region 9 are coplanar. In this state of the diaper 1, the elasticity of the respective elastic members is assumed to be inactive. The diaper 1 has a longitudinal center line P-P bisecting a dimension thereof in the transverse direction X and a transverse center line Q-Q bisecting a dimension thereof in the longitudinal direction Y. The diaper 1 is bilaterally symmetric about the longitudinal center line P-P.

As will be seen in this plan view, the chassis 2 comprises the inner and outer sheets 11, 12 being identical to each other with respect to the shape as well as to the size and contoured by pairs of opposite side edges 14, 14; 15, 15 of the respective waist regions and opposite leg-surrounding peripheral edges 17, 17 extending between these pairs of opposite side edges 14, 14; 15, 15. The pair of leg-surrounding peripheral edges 17, 17 bows convexly toward the longitudinal center line P-P. The inner and outer sheets 11, 12 are formed, for example, by an air-permeable and hydrophobic nonwoven fabric and bonded or fused together through the intermediary of the leak-barrier sheet 13.

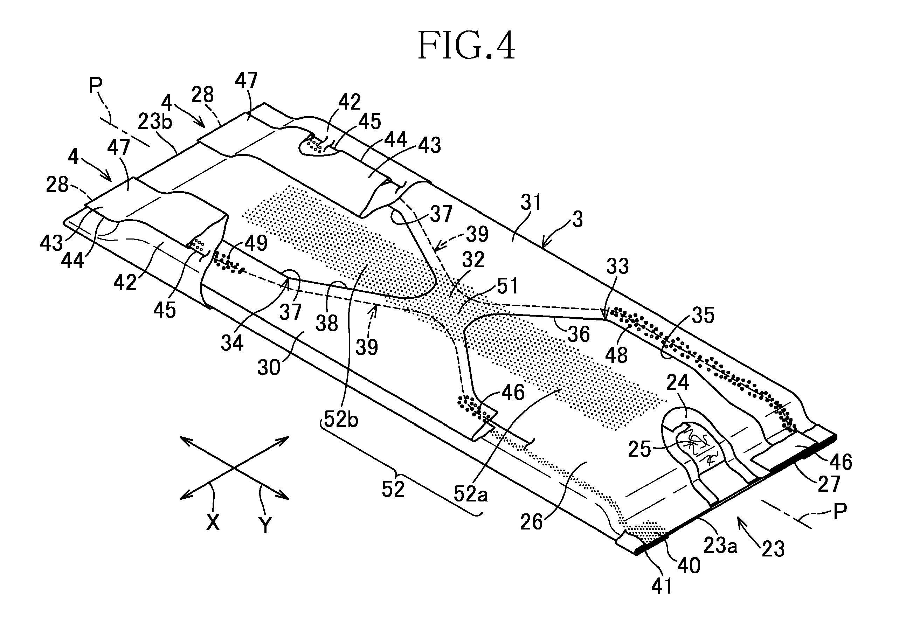

Fig. 4 is a partially cutaway explanatory diagram of the diaper 1 with the inner and outer sheets 11, 12 eliminated so as to be substantially focused on the separator sheet 3 and the liquid-absorbent panel 23. As illustrated, the separator sheet 3 is provided on the inner side of the liquid-absorbent panel 23 facing the wearer's skin and the leak-barrier cuffs 4 are provided on the inner side of the separator sheet 3 facing the wearer's skin.

The separator sheet 3 comprises a pair of lateral zones 30, 31 opposed to each other in the transverse direction X and a middle zone 32 by which these lateral zones 30, 31 are contiguous to each other, wherein the middle zone 32 lies in the crotch region 9. The lateral zones 30, 31 and the middle zone 32 cooperate to form front and rear through-holes 33, 34. The front through-hole 33 for passage of urine extends from the middle zone 32 into the front waist region 7 substantially in U-shape and the rear through-hole 34 for passage of feces extends from the middle zone 32 into the rear waist region 8 substantially in U-shape. The front through-hole 33 for passage of urine is defined by respective inner side edges 35 of the lateral zones 30, 31 and a curved margin of closure 36 making these inner side edges 35 contiguous to each other. The rear through-hole 34 for passage of feces is defined by respective inner side edges 37 of the respective lateral zones 30, 31 and a curved margin of closure 38 making these inner side edges 37 contiguous to each other.

The separator sheet 3 is provided with a pair of separator sheet biasing elastic members 39 extending in the longitudinal direction Y along the front and rear through-holes 33, 34 and attached under tension to the separator sheet 3. The separator sheet biasing elastic members 39 are attached to the separator sheet 3 in a substantially symmetrical relationship about the longitudinal center line P-P and are curved in the vicinity of the middle zone 32 toward the longitudinal center line P-P.

The separator sheet 3 has the opposite lateral zones 30, 31 are folded toward the side of the liquid-absorbent panel 23 in a two-ply fashion. The separator sheet biasing elastic members 39 are sandwiched between these two-ply layers of the lateral zones 30, 31 and bonded thereto by adhesive (not shown). The separator sheet 3 is bonded to the liner 26 facing the wearer's skin of the liquid-absorbent panel 23 by means of adhesive 40 at least at the front and rear ends 27, 28 of the lateral zones 30, 31 folded in a two-ply fashion.

The leak-barrier cuffs 4 comprise a pair of sheets extending in the longitudinal direction Y symmetrically about the longitudinal center line P-P. This sheet may be formed, for example, of a nonwoven fabric or plastic film preferably of liquid-impervious nature. The leak-barrier cuffs 4 are provided so as to overlap the respective lateral zones 30, 31 of the separator sheet 3 on their sides facing the wearer's skin.

Each of the leak-barrier cuffs 4 comprises a three-ply sheet having a Z-shaped cross section. More specifically, the leak-barrier cuff 4 comprises a first layer 41 sandwiched between the liquid-absorbent panel 23 and the chassis 2 and bonded to the inner sheet 11 of the chassis 2, a second layer 42 folded back from the first layer 41 onto the side of the separator sheet 3 facing the wearer's skin so as to be placed upon the lateral zone 30 or 31 and a third layer 43 folded back onto the side of the second layer 42 facing the wearer's skin. The third layer 43 is folded back onto itself along a side edge 44 thereof so that a cuff biasing elastic member 45 is able to be attached thereto. The cuff-biasing elastic member 45 is wrapped with this side edge 44 folded back in this manner and attached under tension thereto so as to extending in the longitudinal direction Y.

At front and rear ends 46, 47 of the leak-barrier cuff 4, the first, second and third layers 41, 42, 43 are bonded one to another and collectively bonded to the inner sheet 11 of the chassis 2. Under contractile force of the cuff biasing elastic member 45 exerted on the leak-barrier cuff 4, the second layer 42 and the third layer 43 are unfolded upward and thereby spaced from the barrier sheet 3 so as to form the wall as seen in Fig. 2. The separator sheet 3 and the leak-barrier cuffs 4 are joined together along front and rear joint zones 48, 49 both provided so as to overlap the separator sheet biasing elastic members 39.

The separator sheet 3 is formed in the middle zone 32 with a first lotion-coated region 51 and the liquid-absorbent panel 23 is formed on the liner 26 facing the wearer's skin with a second lotion-coated region 52. The second lotion-coated region 52 comprises a front lotion-coated sub-region 52a exposed through the front through-hole 33 and a rear lotion-coated sub-region 52b exposed through the rear through-hole 34 wherein the front and rear lotion-coated sub-regions 52a, 52b are separated from each other by the intermediary of the first lotion-coated region 51. The lotion comprises an emollient ingredient and an immobilizing ingredient serving to immobilize the emollient ingredient to the separator sheet and the chassis and is sprayed onto the separator sheet 3 and the liquid-absorbent panel 23. Specifically, spraying of the lotion is carried out with the separator sheet 3 and the leak-barrier cuffs 4 both laminated on the liquid-absorbent panel 23, more specifically, with the stretching force of the respective elastic members being inactivated. The lotion is sprayed from the side of the wearer's skin toward the liquid-absorbent panel 23 so that the first and second lotion-coated regions 51, 52 are formed at once since the separator sheet 3 is laminated on the liquid-absorbent panel 23 during operation of spraying. In this way, the first and second lotion-coated regions 51, 52 are integrally and continuously formed.

The first and second lotion-coated regions 51, 52 extend on the longitudinal center line P-P in the longitudinal direction and totally have a length-dimension of about 50 to 60 mm as measured in the transverse direction X. A length-dimension as measured in the longitudinal direction Y of these lotion-coated regions extending from the front end of the front coated sub-region 52a of the second lotion-coated region 52 across the first lotion-coated region 51 to the rear end of the rear coated sub-region 52b is about 250 to 300 mm. A length-dimension as measured from the front end 27 of the liner 26 facing the wearer's skin to the front end of the front coated sub-region 52a is about 50 mm. Application quantity of the lotion in the first and second lotion-coated regions 51, 52 is about 3 to 15g/m², preferably about 10g/m².

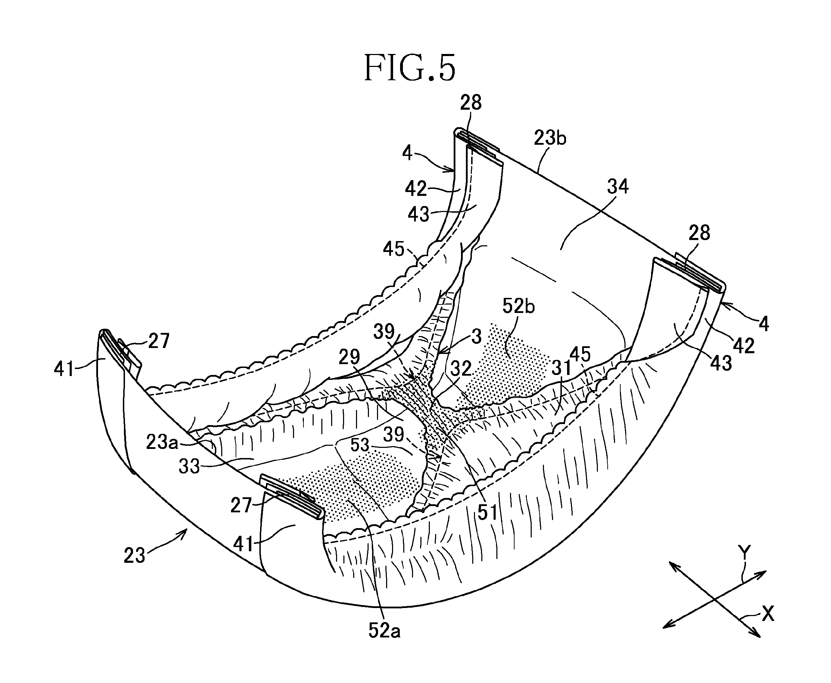

While the respective states of the liquid-absorbent panel 23, the separator sheet 3 and the leak-barrier cuffs 4 have been described above on the assumption that the associated elastic members are inactivated, these states are changed to respective new states on the assumption that the associated elastic members are activated as illustrated by Fig. 5. As illustrated, the liquid-absorbent panel 23 bows and consequently the leak-barrier cuffs 4 are spaced upward from the liquid-absorbent panel 23 under contraction of the separator sheet biasing elastic members 39 and the cuffs biasing elastic members 45. Contraction of the separator sheet biasing elastic members 39 causes the separator sheet 3 to lift toward the wearer's skin with the middle zone 32 thereof being spaced from the liquid-absorbent panel 23. The middle zone 32 is formed with the first lotion-coated region 51 while the liquid-absorbent panel 23 is formed with the second lotion-coated region 52 so that the first and second lotion-coated regions 51, 52 having integrally extended until then are now separated from each other in the direction in which the separator sheet 3 is spaced from the liquid-absorbent panel 23.

Since the separator sheet 3 is held in close contact with the liquid-absorbent panel 23 when the first and second lotion-coated regions 51, 52 are formed, a portion of the liquid-absorbent panel 23 on which the separator sheet 3 has been placed is formed of a lotion-uncoated region 53 which is left uncoated with the lotion. Specifically, the liner 26 facing the wearer's skin is formed in the crotch region 9 with the second lotion-coated region 52 and this lotion-uncoated region 53.

The liner 26 facing the wearer's skin is formed in the crotch region 9 with the lotion-uncoated region 53 in the manner as has been described just above and therefore the liquid-perviousness of the liner 26 facing the wearer's skin is maintained by this lotion-uncoated region 53. Specifically, bodily fluids such as urine can be absorbed by the liquid-absorbent core 25 through the intermediary of the lotion-uncoated region 53 of the liner 26 facing the wearer's skin. The separator sheet 3 spaced from the liquid-absorbent panel 23 is formed in the middle zone 32 thereof with the first lotion-coated region 51 and the middle zone 32 necessarily comes in contact with the wearer's skin. Consequently, the lotion is surely transferred from the first lotion-coated region 51 to the wearer's skin. The diaper 1 is put on the wearer's body so that the front through-hole 33 of the separator sheet 3 is opposed to the wearer's external genital and the rear through-hole 34 of the separator sheet 3 is opposed to the wearer's anus. As a result, the middle zone 32 comes in contact with a region of the wearer's skin extending between the external genital and the anus. This region of skin is sensitive to irritation and apt to suffer from skin troubles such as eruption. The lotion transferred from the first lotion-coated region 51 to such region of skin sensitive to irritation serves to protect the wearer's skin from various skin troubles even in the unlikely event that the wearer's skin may be soiled with body waste such as feces.

The lotion-uncoated region 53 is formed on the side of the first lotion-coated region 51 facing the wearer's clothes and obviously out of contact with the wearer's skin so long as the void space 29 is maintained during use of the diaper 1. Therefore, the lotion-uncoated region 53 is prevented from contacting with the wearer's skin and causing various skin troubles. Even if the void space 29 is collapsed, for example, as the wearer sits down and, in consequence, the separator sheet 3 is put flat together with the liquid-absorbent panel 23, the presence of the first lotion-coated region 51 interposed between the lotion-uncoated region 53 and the wearer's skin prevents the lotion-uncoated region 53 from coming in direct contact with the wearer's skin.

While the process of lotion coating is assumed to be carried out by spraying as far as the present embodiment is concerned, any other methods or devices, for example, a slot coater may be used for this process. In any case, the first lotion-coated region 51 and the second lotion-coated region 52 may be simultaneously formed to reduce the time taken for manufacturing and thereby the manufacturing cost which would otherwise significantly increase for the process of lotion coating can be avoided. The lotion consisting of the emollient ingredient and the immobilizing ingredient adapted to immobilize the emollient ingredient to the separator sheet and the chassis effectively functions to emollient the wearer's skin and to alleviate a possibility the wearer suffer from any skin troubles. While the separator sheet biasing elastic members 39 are bonded to the separator sheet 3 by means of adhesive, the adhesive might come off due to the lotion contained in the middle zone 32 of the separator sheet 3. To overcome this problem, the quantity of adhesive used to bond the elastic members 39 to the separator sheet 3 is preferably increased particularly in the middle zone 32.

The separator sheet 3 as well as the leak-barrier cuffs 4 may be formed, for example, of a liquid-resistant/ air-permeable nonwoven fabric, the inner sheet 11 as well as the outer sheet 12 may be formed, for example, of an air-permeable nonwoven fabric, the leak-barrier sheet 13 may be formed, for example, of a moisture-pervious plastic film, and the liquid-absorbent panel 23 may be formed, for example, of a mixture of fluff pulp and super-absorbent polymer particles. In general, various stock materials conventionally used in the relevant technical field may be used for these components.

### <Second Embodiment>

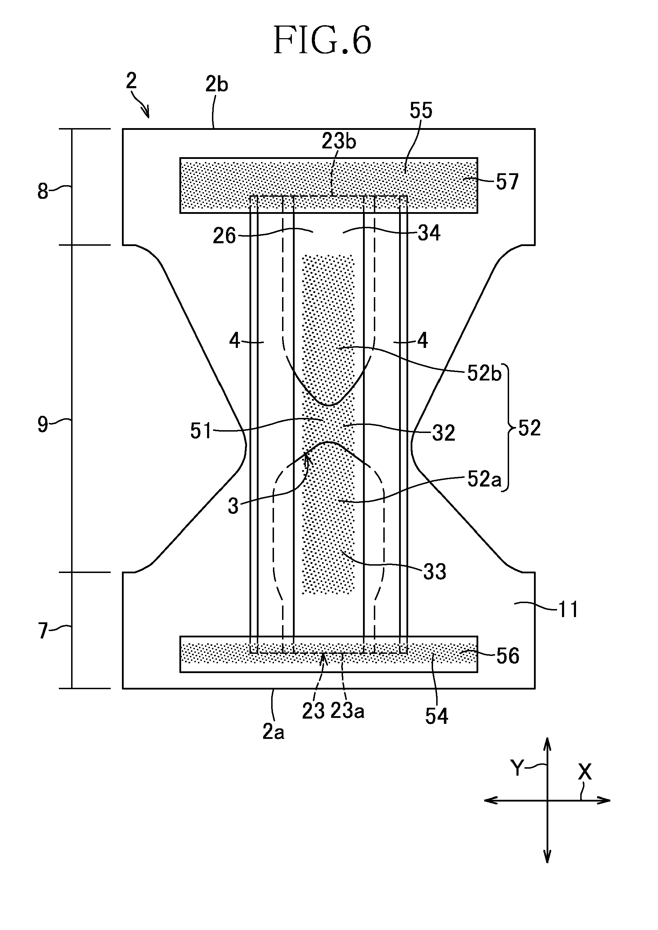

Fig. 6 is a view similar to Fig. 3 of the first embodiment, illustrating a second embodiment. In this second embodiment, constituent elements similar to those in the first embodiment will not be repetitively described. According to this second embodiment, the front and rear waist regions 7, 8 are provided with front and rear waist sheets 54, 55 which are, in turn, coated with the lotion further to define a third front lotion-coated region 56 and a third rear lotion-coated region 57. The front and rear waist sheets 54, 55 extend in the transverse direction X substantially in parallel to the front and rear ends 2a, 2b of the chassis 2 cooperating together to form the waist-opening 18. The front and rear waist sheets 54, 55 are placed aside in the longitudinal direction Y so as to overlap the front and rear ends 23a, 23b of the liquid-absorbent structure 23, respectively, but not to overlap the liquid-absorbent core 25.

The front and rear waist sheets 54, 55 may be formed, for example, of a water-repellent or hydrophilic nonwoven fabric of conventional type or a nonwoven fabric impregnated with antibacterial agent. The front and rear waist sheets 54, 55 are wholly coated with the lotion to form third front and rear lotion-coated regions 56, 57. Application quantity of the lotion is about 3 to 15 g/m², preferably about 10 g/m².

During use of the diaper 1, the front and/or rear waist regions 7, 8 often remain in contact with the ventral region and/or the dorsal region, respectively, of the wearer. Accordingly, when the front and rear waist regions 7, 8 are provided with the front and rear waist sheets 54, 55, respectively, these front and rear waist sheets 54, 55 will come in contact with the wearer's skin. These front and rear waist sheets 54, 55 may be coated with the lotion to define the third front and rear lotion-coated regions 56, 57 and thereby to prevent the wearer from suffering from skin troubles due to friction with the diaper 1. Furthermore, the unique placement of these front and rear waist sheets 54, 55 so as not to overlap the liquid-absorbent core 25 assures that the third front and rear lotion-coated regions 56, 57 defined by the front and rear waist sheets 54, 55 never disturb absorption of bodily fluid by the liquid-absorbent core 25.

According to this second embodiment, the third front and rear lotion-coated regions 56, 57 are provided, in addition to the first and second lotion-coated regions 51, 52 and thereby the wearer can be protected from suffering from any skin trouble more reliably. While the third front and rear lotion-coated regions 56, 57 are formed by the front and rear waist sheets 51, 52 provided separately of the chassis 2 according to this second embodiment, it is also possible to form the third front and rear lotion-coated regions 56, 57 directly on the chassis 2.

## Claims

1. An absorbent article comprising a chassis 2 having longitudinal and transverse directions, sides facing the wearer's skin and a garment of a wearer 5, 6, respectively, a front waist region 7, a rear waist region 8, a crotch region 9 continuously extending between said front and rear waist regions, and a liquid-absorbent structure 10 provided on at least said crotch region, and a separator sheet 3 provided on said side of said chassis facing said wearer's skin and adapted to be spaced from said chassis in said crotch region, wherein said chassis is formed with a waist-opening 18 in said front and rear waist regions and with a pair of leg-openings 19 in said crotch region, said absorbent article being **characterized in that**:
said separator sheet comprises lateral zones 30, 31 extending said longitudinal direction and opposite in said transverse direction, a middle zone 32 connected between said lateral zones, through-holes defined by said lateral zones and said middle zone so as to communicate a side facing the wearer's skin with a side facing said chassis, separator sheet biasing elastic members 21 extending in said longitudinal direction and attached under tension to said separator sheet, and front and rear ends opposite in said longitudinal direction and extending in said transverse direction along which said separator sheet is permanently bonded to said side of said chassis facing the wearer's skin so that a void space 29 is formed between said separator sheet and said liquid-absorbent structure and at least said middle zone comes in contact with the wearer's skin as said liquid-absorbent structure bows about said crotch region so that said front and rear waist regions are opposed to each other; and said separator sheet is coated in said middle zone with lotion so as to form a first lotion-coated region 51 and said chassis is coated on portions exposed through said through-holes with said lotion so as to form a second lotion-coated region 52.

2. The absorbent article according to Claim 1, wherein said chassis is provided in said crotch region with a lotion-uncoated region 53 which is left not coated with said lotion and said lotion-uncoated region is formed in a region of said chassis opposed to said middle zone of said separator sheet.

3. The absorbent article according to Claim 1 or 2, wherein said lotion is applied from said side of said separator sheet facing the wearer's skin toward said chassis.

4. The absorbent article according to any one of Claims 1 through 3, wherein said separator sheet includes a front through-hole 33 lying on a side of said front waist region and a rear through-hole 34 lying on a side of said rear waist region and said middle zone provided in said crotch region.

5. The absorbent article according to any one of Claims 1 through 4, further including a third lotion-coated region formed on said side of said chassis facing the wearer's skin in at least one of said front and rear waist regions.

6. The absorbent article according to any one of Claims 1 through 5, wherein said third lotion-coated region is formed so as to extend in said transverse direction along said waist-opening.

7. The absorbent article according to any one of Claims 1 through 6, wherein said lotion consists of an emollient ingredient and an immobilizing ingredient serving to immobilize said emollient ingredient to said separator sheet and said chassis.
